# EUROPÄISCHE PATENTSCHRIFT

(11) **EP 0 980 676 B1**
(45) Veröffentlichungstag und Bekanntmachung des Hinweises auf die Patenterteilung: **06.11.2002**
(21) Anmeldenummer: 99116025.0
(22) Anmeldetag: 16.08.1999
(51) Int. Cl.: A61B 17/115

(54) **Instrument zur chirurgischen Anastomose**
Surgical anastomosis instrument
Dispositif chirurgical d'anastomose

(30) Priorität: 17.08.1998 DE 19837258
(43) Veröffentlichungstag der Anmeldung: 23.02.2000
(73) Patentinhaber: Deutsches Zentrum für Luft- und Raumfahrt e.V., 53175 Bonn (DE)
(72) Erfinder: Balazs, Matthias, 82284 Grafrath (DE); Hagn, Ulrich, 82234 Wessling (DE)
(74) Vertreter: von Kirschbaum, Albrecht, Dipl.-Ing.

(56) Entgegenhaltungen:
- EP-A- 0 152 382
- EP-A- 0 399 701
- WO-A-82/00968
- DE-A- 19 509 115
- US-A- 5 533 661

## Beschreibung

Die Erfindung betrifft eine Vorrichtung zum Betätigen von verschiedenenartigen, gegeneinander austauschbaren chirurgischen Instrumenten, insbesondere zur Anastomose (d.h. zum Trennen, Verschließen und Verbinden) von Hohlorganen.

Wie aus DE 195 09 115 A1 bekannt, ist am proximalen Ende eines Schaftteils einer derartigen Vorrichtung ein Griffteil lagerichtig positioniert. Mit dem proximalen Ende eines im Schaftteil geführt untergebrachten Schub- und Zugteils steht ein Kniehebel-Mechanismus in Wirkverbindung, dessen proximales Ende mit einem Verstellmechanismus im Griffteil verbunden ist. Am distalen Ende des Kniehebel-Mechanismus ist ein Anschlußteil zum Herstellen einer Verbindung zu einem chirurgischen Instrument vorgesehen. Am distalen Ende des Schaftteils ist ferner ein Anschluß zum Anschließen eines Gehäuses eines chirurgischen Instruments vorgesehen, während am distalen Ende des flexiblen Schub- und Zugteils ein axiale Druckkraft an den Körper des chirurgischen Instruments übertragendes Schubteil angebracht ist.

Aus US 4,573,468 ist ein chirurgisches Nahtklammergerät mit einer wiederverwendbaren Einheit aus Griff- und Schaftteil sowie einer aufsteckbaren Einweg-Kopfeinheit bekannt. Dieses Gerät weist einen pistolenartigen Griffteil und einen geraden Schaftteil auf, die nicht voneinander trennbar sind. Der Schaftteil ist als starres, gerades Rohr ausgeführt.

Eine aufsteckbare Einweg-Kopfeinheit in Form des Nahtklammergeräts, welches Nahtklammern, einen Klammerausstoßer, ein Zirkularskalpell, einen Verbindungsstift sowie eine Andruckplatte aufweist, wird am distalen Ende des Schaftteils mittels eines Bajonettverschlusses bzw. einer Verschraubung befestigt.

Aus US 5,533,661 ist ein Nahtklammergerät bekannt, bei welchem weder der Schaft vom Griffteil noch der Kopf vom Schaftteil getrennt werden können. Lediglich eine Andrückplatte und ein Stift können vom Schaftteil getrennt werden. Ferner ist der Griffteil nicht demontierbar und das gesamte Instrument ist ausschließlich zum einmaligen Gebrauch bestimmt.

Die bekannten Nahtklammergeräte weisen ähnlich wie andere nicht wiederverwendbare chirurgische Instrumente verschiedene Nachteile auf, die sich in der Praxis als störend erwiesen haben, obwohl nicht jedes dieser Geräte bzw. Instrumente alle nachstehend aufgeführten Nachteile, jedoch meistens mehrere dieser Nachteile gleichzeitig aufweist.

Die bekannten Instrumente haben im allgemeinen ein zu hohes Gewicht. Manche dieser Geräte benötigen zu ihrer Betätigung zu hohe Bedienkräfte und/oder verfügen nicht über eine Rückmeldeeinrichtung, beispielsweise in der Form "Klammerung bzw. Schnitt hat stattgefunden". Bei nicht wiederverwendbaren zirkularen Geräten fallen allein schon deshalb hohe Kosten an, da sie nur einmal benutzt werden können und dann entsorgt werden müssen.

Aufgabe der Erfindung ist es daher, eine Vorrichtung zum Betätigen von chirurgischen Instrumenten so auszuführen und auszulegen, dass sie bei einem möglichst geringen Gewicht mit möglichst wenig Kraft zu bedienen, zum Reinigen in einzelne Gruppen zu zerlegen ist und somit wieder zu verwenden ist.

Zur Lösung dieser Aufgabe ist gemäß der Erfindung bei einer Vorrichtung gemäß dem Oberbegriff des Anspruchs 1 zum lösbaren Verbinden des Schaftteils mit dem Griffteil auf einem Anschlußteil des Schaftteils eine frei drehbare Befestigungsmutter vorgesehen, wobei das Anschlussteil einen gemeinsamen Anschlag für Befestigungsmutter und Griffgehäuse aufweist und der Kniehebelmechanismus aus einem zwischen Lagerböcken schwenkbar gelagerten ersten Schenkel und einem mit dem ersten Schenkel beweglich verbundenen zweiten Schenkel dadurch lösbar mit dem proximalen Ende des Schub- und Zugteils in Wirkverbindung steht, dass in einem Anschlussteil am proximalen Ende des Schub- und Zugteils eine aus Quer- und Längsnut gebildete Kreuznut vorgesehen ist, welcher im Griffgehäuse und in dem Anschlussteil der Kreuznut entsprechende ausgebildete T-förmige Fenster zugeordnet sind.

Bei der Erfindung lassen sich zum Zwecke der Reinigung/Sterilisation somit in vorteilhafter Weise der Griffteil vom Schaftteil ohne Werkzeug lösen und der Kniehebel-Mechanismus ohne Werkzeug demontieren.

Bei der erfindungsgemäßen Vorrichtung ermöglicht ein Verstellmechanismus eine zügige Annäherung beispielsweise einer Andruckplatte an ein Klammermagazin und darüber hinaus anschließend eine Feineinstellung beispielsweise eines Gewebespaltes. Während der Annäherung, d.h. Bewegung der Andruckplatte wird eine axiale Verstellung durchgeführt, die über ein inneres Schubmittel zu einem Bedienteil am Griffteil übertragen wird. Zur Feineinstellung ist eine Mechanik vorgesehen, durch welche diese axiale Bewegung in eine Drehbewegung umgesetzt wird. Die Drehbewegung wird während eines Eingriffs gesperrt, wodurch dann die axiale Lage des Verstellmechanismus fixiert ist.

Somit ist bei der erfindungsgemäßen Vorrichtung der Verstellmechanismus im Feineinstellbereich automatisch arretiert und kann sich während der Durchführung eines Eingriffs, beispielsweise eines Klammerungsvorgangs nicht verstellen. Durch eine Formsperrung der Drehbewegung kann eine sehr feine Rasterung des Feineinstellbereiches erreicht werden.

Der Schaftteil der erfindungsgemäßen Vorrichtung weist eine Mechanik auf, um eine bestimmte Kraft, beispielsweise bei einem Klammerungsvorgang, sowie einen vorgegebenen Weg, wie beispielsweise einen Verstellweg für eine Andruckplatte weiterzuleiten. Hierbei können die Kraft und der Verstellweg sowohl bei geraden als auch bei einachsig gebogenen Schaftrohren aufgebracht bzw. ausgeführt werden.

Die erfindungsgemäße Vorrichtung besteht lediglich aus zwei Baugruppen, die aus wenigen wiederholt verwendbaren, einfach und verdrehsicher montierbaren und wieder demontierbaren Elementen bestehen, die jeweils leicht zu reinigen und damit leicht wieder zu sterilisieren sind. Über einen leicht zu bedienenden Druckknopf- bzw. Aufsteckanschluß können an die Vorrichtung gemäß der Erfindung unterschiedliche Kopfeinheiten angeschlossen werden.

Nachfolgend wird die Erfindung anhand bevorzugter Ausführungsbeispiele unter Bezugnahme auf die anliegenden Zeichnungen im einzelnen erläutert. Es zeigen:
- Fig.1a und 1b: eine schematische, perspektivische Ansicht einer bevorzugten Ausführungsform eines Griffteils sowie eines davon gelösten Schaftteils;
- Fig.2a: in unmaßstäblicher Darstellung eine Draufsicht einer Mechanik im Schaftteil sowie
- Fig.2b: eine vergrößerte Detaildarstellung eines Teilbereichs der Fig.2a;
- Fig.3: schematisch einen Verstellmechanismus;
- Fig.4: eine axiale Schnittansicht des an dem Schaftteil befestigten Griffteils bei betätigtem Bedienteil;
- Fig.5: eine der Fig.4 entsprechende axiale Schnittansicht des an dem Schaftteil befestigten Griffteils bei nicht betätigtem Bedienteil;
- Fig.6: eine perspektivische Ansicht der miteinander verbundenen Griff- und Schaftteile und einer schematisch angedeuteten, montierten Einweg-Kopfeinheit;
- Fig.7a: eine axiale Schnittansicht durch den distalen Endteil des Schaftteils mit einem Anschluß für einen Verbindungsstift;
- Fig.7b: eine vergrößerte Darstellung eines Details des Endteils der Fig.7a;
- Fig.7c: eine perspektivische Darstellung des Endteils des Schaftteils und einer Aufnahme des Verbindungsstiftes,
- Fig.7d: eine vergrößerte perspektivische Darstellung eines Druckknopf-Anschlusses am distalen Ende des Schaftteils mit einem diesem zugeordneten Druckknopfmechanismus am proximalen Ende eines Klammermagazins,
- Fig.7e: eine modifizierte Ausführung des distalen Endes des Schaftteils und ein diesem angepaßtes proximale Ende eines Klammermagazins;
- Fig.8a: ein gerade ausgerichtetes, verformbares Schaftrohr und gestrichelt ein nach rechts gebogenes Schaftrohr;
- Fig.8b: im Schnitt eine vergrößerte Darstellung eines Ausschnittes eines distalen Schaftrohrendes, und
- Fig.8c: eine perspektivische Explosionsdarstellung von Einzelteilen zum Steuern des Schaftrohrs.

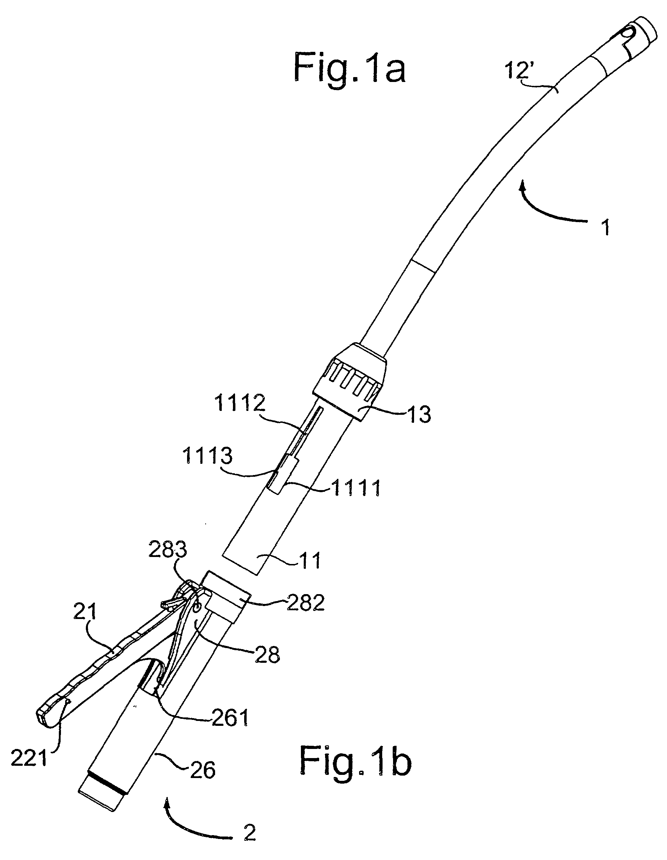

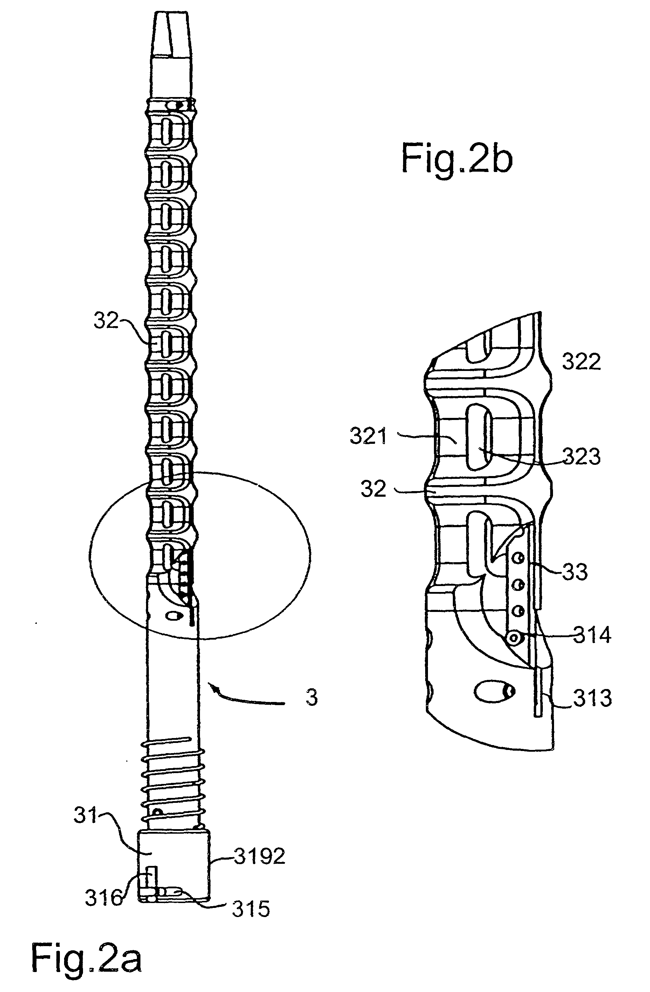

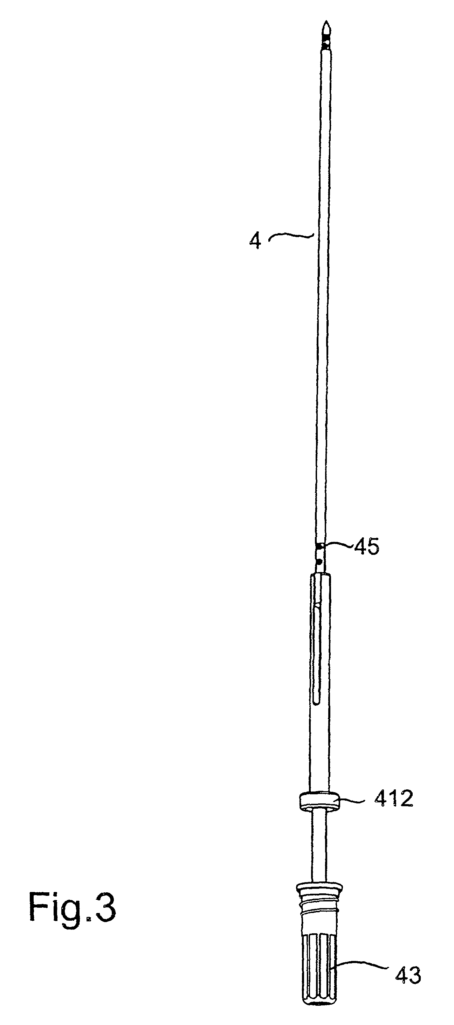

In Fig.1a bis Fig.3 sind schematisiert in teilweise perspektivischer Darstellung die Baugruppen Schaftteil 1 (Fig.1a), Griffteil 2 (Fig.1b), Mechanik 3 im Schaftteil 1 (Fig.2a und 2b) und Verstellmechanismus 4 (Fig.3) wiedergegeben.

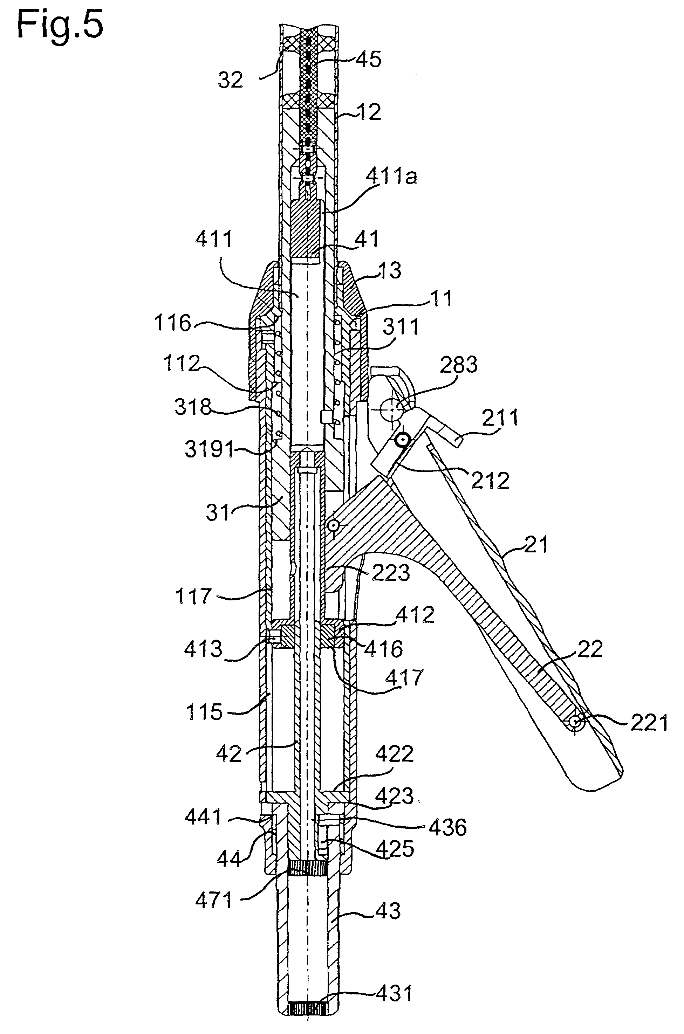

Nachstehend werden als erstes die verschiedenen Elemente des Griffteils 2 sowie des Schaftteils 1 beschrieben. Das Griffteil 2 weist ein rohrförmiges Gehäuse 26, einen schwenkbaren ersten Schenkel 21 sowie einen zweiten Schenkel 22 (Fig.5) auf; hierbei bilden der erste Schenkel 21 und der zweite Schenkel 22 zusammen einen Kniehebel.

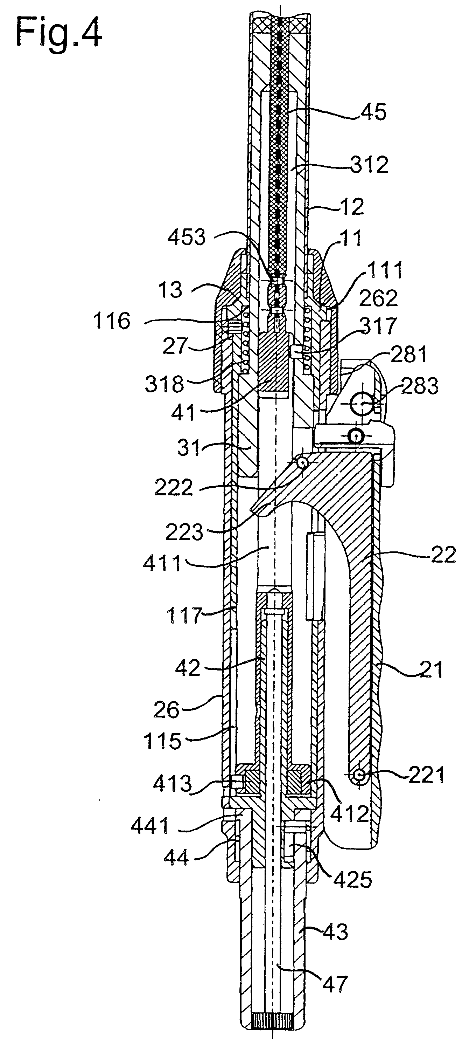

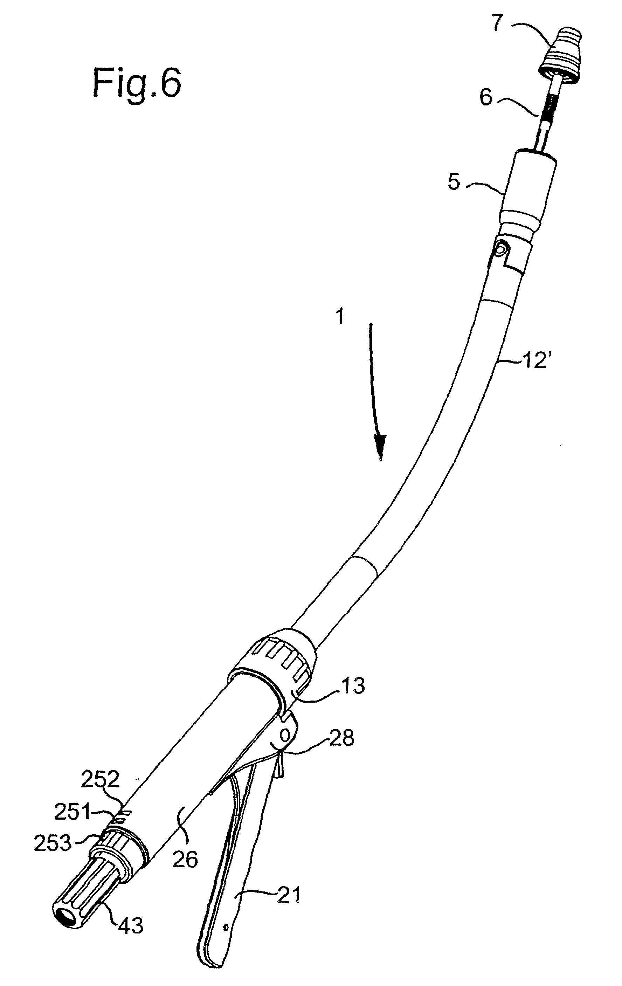

Wie in Fig.6 dargestellt, sind am Gehäuse 26 eine als Durchbruch ausgeführte Anzeige 252 zur Verifikation, ob sich der Verstellmechanismus 4 im Feineinstellbereich befindet, eine Kontrollanzeige 251 bezüglich einer Arretierung des Verstellmechanismus, und eine Skala 253 für eine eingestellte Spaltbreite vorgesehen. Darüber hinaus ist am Gehäuse 26, um eine lagerichtige Montage des Schaftteils 1 zu gewährleisten, an dessen distalen Ende eine Montagenut 27 (Fig.4) ausgebildet.

Der ersten Schenkel 21 ist mittels eines Bolzens 283 in zwei Lagerböcken 28 (Fig.6) schwenkbar gehalten. Der zweite Schenkel 22 ist über einen Bolzen 221 mit ersten Schenkel 21 beweglich verbunden (Fig.4 und 5). An dem dem Bolzen 221 abgewandten Ende weist der zweite Schenkel 22 einen Querstift 222 auf (Fig.4), durch den eine Verbindung mit der Baugruppe Mechanik 3 über eine durch eine Quernut 315 und eine Längsnut 316 gebildete Kreuznut (Fig.2a) mit einem Anschlußteil 31 eines flexiblen Schub- und Zugteils 32 hergestellt ist.

Durch im Gehäuse 26 des Griffteils 2 und äquivalent in einem Anschlußstück 11 des Schaftteils 1 ausgebildete, T-förmige Fenster 261 bzw. 1111 (siehe Fig.1a und 1b) kann beim Montieren bzw. Demontieren der mit dem zweiten Schenkel 22 fest verbundene Querstift 222 in die Quernut 315 eingeführt bzw. aus dieser herausgenommen werden.

Durch die T-förmigen Fenster 261, 1111 kann der zweite Schenkel 22 mit seinem Querstift 222 genau dann in die Quernut 315 des Anschlußteils 31 eingesetzt werden, wenn der Kniehebel 21, 22 maximal geöffnet ist. Während des Betriebes verhindert jeweils ein schmaler Bereich 1112 des T-förmigen Fensters 1111, daß der zweite Schenkel 22 unbeabsichtigt aus dem Anschlußteil 31 herausspringt.

Der Kniehebel 21, 22 kann beim Montieren bzw. Demontieren nur dann weiter als in Fig.5 dargestellt, geöffnet werden, wenn bezüglich einer Aussparung 281 (Fig.4) eine Befestigungsmutter 13 nicht auf ein Gewinde 262 des Gehäuses 26 geschraubt ist. Dadurch kann bei der Montage/Demontage der Kniehebel 21, 22 so ausgerichtet werden, daß der Querstift 222 innerhalb eines breiten Bereichs 1113 des T-förmigen Fensters 1111 zu liegen kommt und dadurch der Kniehebel 21, 22 vom Anschlußteil 31 des flexiblen Schub- und Zugteils 32 getrennt werden kann.

Über den Bolzen 283 ist der erste Schenkel 21 beweglich bzw. schwenkbar in den Lagerböcken 28 des Gehäuses 26 gehalten, zwischen denen sich somit Schenkel 21 und Schenkel 22 des Kniehebels bewegen können. Gleichzeitig ist es aufgrund der Formgebung der Lagerböcke 28, des ersten Schenkels 21 und des zweiten Schenkels 22 ausgeschlossen, daß versehentlich zwischen Schenkel 21 und Schenkel 22 gegriffen werden kann.

Im ersten Schenkel 21 ist eine Sperre 211 (Fig.5) integriert, die ein unbeabsichtigtes Betätigen des Kniehebels verhindert. Beispielsweise nach Durchführen einer Klammerung und Erreichen der in Fig.5 dargestellten Kniehebelstellung wird durch eine Schenkelfeder 212 die Sperre 211 erneut eingerastet.

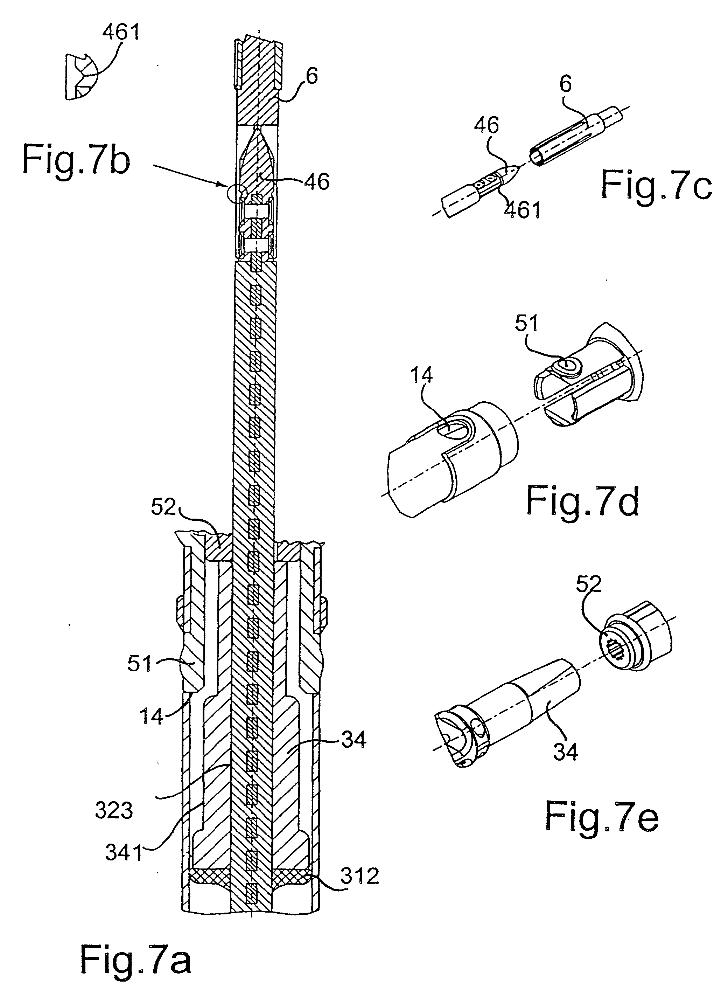

Die Baugruppe Schaftteil 1 ist am proximalen (d.h. dem Bediener zugewandten) Ende mit der Baugruppe Griffteil 2 fest verbindbar und am distalen (d.h. vom Bediener abgewandten) Ende kann beispielsweise ein in Fig.6 nur schematisch angedeutetes und nicht näher beschriebenes Klammermagazin 5 mit an dessen proximalen Ende vorgesehenen Schnappnasen 51 eines Druckknopfmechanismus über einen Druckknopfanschluß 14 angebracht werden, was in Fig.7d vergrößert wiedergegeben ist.

Die Baugruppe Schaftteil 1 kann ein verformbares, gebogenes Schaftrohr 12' (Fig.1a und 6) oder auch ein gerades starres Schaftrohr 12 (Fig.4 und 5) aufweisen und hat ferner ein mit dem jeweiligen Schaftrohr 12 bzw. 12' verbundenes Anschlußstück 11 und die auf diesem frei drehbare Befestigungsmutter 13. Das Anschlußstück 11 weist einen Anschlag 111 (Fig.4) sowohl für die Mutter 13 als auch für das Gehäuse 26 auf.

Zur Führung und Verdrehsicherung eines Mittelteils 41 des Verstellmechanismus 4 mittels eines von diesem vorstehenden Bolzens 413 ist eine Längsnut 115 im Schaftteil 1 vorgesehen. In einer rohrförmigen Laufbahn 117 ist der Mittelteil 41 durch einen kreiszylindrischen Ansatz 412 (Fig.5) und der Anschlußteil 31 durch einen kreiszylindrischen Abschnitt 3192 (Fig.2a) koaxial geführt.

Die zum Weiterleiten bespielsweise einer Kraft und einer Axialbewegung entlang des Schaftrohres 12 vorgesehene Baugruppe Mechanik 3 besteht aus insgesamt drei Hauptkomponenten: dem Anschlußteil 31, das überwiegend im Griffteil 2 untergebracht ist, dem flexiblen Schub- und Zugteil 32 und zwei in diesem eingebetteten dünnen Metallkörpern 33 (Fig.2b), die durch ihre axiale Steife eine Axial-Kraft weiterleiten. Der flexible Schub- und Zugteil 32 führt die Metallkörper 33 entlang der neutralen Faser und läßt gleichzeitig aufgrund seiner Aussparungen 321 und kreiszylindrischen Abschnitte 322 aufweisenden Mantelfläche eine einachsige Biegung zu.

Die Kraft wird am distalen Ende der Baugruppe Mechanik 3 durch ein abgestuftes Schubteil 34 (Fig.7b und 7c) als axiale Druckkraft an ein korrespondierendes Bauteil beispielsweise in einem Klammermagazin 5 übertragen. Das Schubteil 34 weist an seinem distalen Ende einen Abschnitt 341 mit einem entsprechend großen Durchmesser auf, so daß beim Ausführen beispielsweise einer Klammerung durch Vorschieben des Schubteils 34 und des damit in Verbindung stehenden flexiblen Schub- und Zugteils 32 in distaler Richtung die Schnappnasen 51 des Druckknopfanschlusses 14 (siehe Fig.7d) gegen unbeabsichtigtes Öffnen gesichert werden. Die Baugruppe Mechanik 3 ist durchgehend rohrförmig ausgebildet und weist mindestens einen vorzugsweise zentralen (entlang der neutralen Faser geführten) Arbeitskanal 312 bzw. 323 auf, in welchem ein Teil des Verstellmechanismus 4 beweglich geführt ist.

Auf der zylindrischen Außenfläche 311 des Anschlußteils 31 ist eine Feder 318 geführt (Fig.4 und 5). Durch einen Hohlstift 317 und eine Nut 411a (Fig.5) sind die Baugruppen Mechanik 3 und Verstellmechanismus 4 zueinander rotatorisch ausgerichtet. Die komplette Baugruppe Mechanik 3 ist gegen eine ringförmige Anschlagfläche 116 mittels der Feder 318 vorgespannt, so daß der Kniehebel 21, 22 nach Durchführen beispielsweise einer Klammerung wieder in die in Fig.5 abgebildete Ausgangsstellung zurückgebracht wird.

Zum Bewegen der Baugruppe Mechanik 3 gegenüber den Baugruppen Schaftteil 1 und Griffteil 2 greift der Querbolzen 222 des Schenkels 22 in die Quernut 315 des Anschlußteils 31 ein. Dadurch wird die vom Kniehebel 21, 22 erzeugte Axialkraft an die Baugruppe Mechanik 3 weitergeleitet.

Im großvolumigen Arbeitskanal 312 gleitet der Mittelteil 41. Eine Anschlagfläche 3191 (Fig.5) des Anschlußteils 31 bildet mit einer Anschlagfläche 112 des Anschlußteils 11 die Wegbegrenzung der Mechanik 3. Eine Nut 313 (Fig.2b) nimmt die kraftübertragenden Metallkörper 33 auf, die durch Nieten 314 mit dem Anschlußteil 31 fest verbunden sind. Die Metallkörper 33 sind im flexiblen Schub- und Zugteil 32, eingebettet und sind dadurch entlang der neutralen Faser des Schaftrohres 12 geführt.

Der flexible Schub- und Zugteil 32 weist den vorzugsweise zentral angeordneten Arbeitskanal 323, Aussparungen 321, die es ermöglichen, den flexiblen Schub- und Zugteil 32 entsprechend der Schaftrohrbiegung elastisch zu verformen, und kreiszylindrische Abschnitte 322 auf, welche einerseits zur Führung sowohl in einem geraden als auch in einem gebogenem Schaftrohr dienen und andererseits ein Ausknicken der Metallkörper 33 verhindern. Durch die Perforation der Metallkörper 33 ist eine gute Verbindung mit dem flexiblen Schub- und Zugteil 32 gewährleistet.

Es ist auch möglich, auf die Metallkörper 33 zu verzichten und eine Kraft ausschließlich über den flexiblen Schub- und Zugteil 32 zu übertragen. Vorzugsweise entlang der neutralen Faser des gebogenen Schaftrohres 12 können wahlweise noch weitere Arbeitskanäle im flexiblen Schub- und Zugteil 32 ein gearbeitet sein, um die Mechanik gegebenenfalls um zusätzliche Funktionen zu erweitern.

Durch den Verstellmechanismus 4 ist für einen Bediener am Griffteil 2 ein Bedienelement geschaffen, mit dessen Hilfe beispielsweise eine Andruckplatte 7 ein- bzw. ausgefahren werden kann. Eine Spindel 42 ist rotatorisch beweglich, aber durch Anschläge 422 und 423 (Fig.5 unten) gegenüber dem Anschlußstück 11 und dem Gehäuse 26 axial festgelegt in dem Griffteil 2 gelagert. Die Spindel 42 ist in einen Gewindeeinsatz 416 des Mittelteils 41 eingeschraubt.

Über Nieten 453 (Fig.4) ist an das Mittelteil 41 als Schubmedium eine Seele 45 angeschlossen, die aus einem kraftübertragenden Metallkörper besteht, der in einem flexiblen, vorzugsweise aus Kunststoff hergestellten, zylindrischen Körper zu Führungs- und Schutzzwecken eingebettet ist. Mittels der Seele 45 wird der Verstellweg von dem Griffteil 2 entlang des Schaftrohres 12 über die umlaufende Nut 461 (Fig.7b) am Anschluß 46 an, beispielsweise einen Verbindungsstift 6 und die Andruckplatte 7 weitergeleitet.

Das Mittelteil 41 ist rotatorisch durch den Bolzen 413 und die Nut 115 gegenüber dem Anschluß 11 festgelegt. Das Bedienmoment für den Verstellmechanismus 4 wird von einem Handrad 43 über einen Stift 436 und eine Nut 425 auf die Spindel 42 übertragen. Stift 436 und Nut 425 lassen es jedoch zu, das Handrad 43 gegenüber der Spindel 42 um einen bestimmten Weg axial zu verschieben. Dieser Bewegung wirkt eine am Gehäuse 26 abgestützte (nur durch eine Windung angedeutete) Druckfeder 44 entgegen. Durch Drehen am Handrad 43 wird eine translatorische Bewegung des Mittelteils 41 erzeugt, wodurch beispielsweise eine Andruckplatte verstellt wird.

Im letzten Annäherungsbereich beispielsweise der Andruckplatte 7 an ein Klammermagazin 5 wird der Bolzen 413 im Anzeigefenster 252 (Fig.6) sichtbar, wodurch beispielsweise "Klammerungsbereich" angezeigt wird. Eine Verzahnung 471 eines fest mit dem Mittelteil 41 verbundenen Riegels 47 greift in die korrespondierende Verzahnung 431 des Handrades 43 ein. Dadurch ist der Verstellmechanismus 4 und somit beispielsweise die Andruckplatten-Verstellung gesperrt.

Zur weiteren Verstellung muß das Handrad 43 entgegen der Kraft der Druckfeder 44 aus dem Gehäuse 26 herausgezogen werden, so daß die Verzahnungen 431 und 471 nicht mehr in Eingriff sind. Nun läßt sich das Handrad 43 wieder drehen und es kann beispielsweise eine korrekte Spaltbreite über die Skala 253 (Fig.6) eingestellt werden. Sobald eine gewünschte Spaltbreite eingestellt ist, kann das Handrad 43 freigegeben werden, welches unter der Wirkung der Druckfeder 44 ins Gehäuse 26 zurückschnappt. Die Verzahnungen 431 und 471 arretieren dann wieder den Verstellmechanismus 4 und nur bei korrekter Arretierung ist ein Absatz 441 des Handrades 43 im Gehäusefenster 251 (Fig.6) zu sehen.

Der Verstellweg ist begrenzt durch den Anschlag von Flächen 422 und 417 sowie durch die Länge der Nut 115. Erst wenn sich der Verstellmechanismus im Feineinstellbereich befindet, kann der Kniehebel 21, 22 bedient werden, da dann erst ein Anschlag 223 des Schenkels 22 durch einen Schlitz 411 des Mittelteils 41 durchschwenken kann.

Zum Anschließen einer Kopfeinheit, beispielsweise eines zirkularen Nahtklammergeräts, an den Schaftteil 1 der erfindungsgemäßen Vorrichtung sind folgende Einzelanschlüsse vorgesehen:
1. Der Druckknopfanschluß 14 (Fig.7d) verbindet das Gehäuse einer Kopfeinheit über an Biegelaschen sitzende Druckknöpfe 51 mit dem Schaftrohr 12 des Schaftteils 1. Dabei greifen die Druckknöpfe 51 in korrespondierende Radialbohrungen im Schaftrohr 12 des Schaftteils 1 ein.
2. Ein aufsteckbarer Anschluß 46 verbindet einen Körper 52 der Kopfeinheit mit dem Verstellmechanismus 4. Dabei greift ein umlaufender Absatz der Innenkontur des geschlitzten, rohrförmigen Endes des Körpers 6 in eine korrespondierende umlaufende Nut 461 am Anschluß 46.
   Wenn der Verbindungsstift auf den Anschluß 46 aufgesteckt ist und zum Instrument hin verfahren wird, erfolgt eine Sicherung gegen ein unbeabsichtigtes Lösen dadurch, daß das abgestufte Schubteil 34 durch eine entsprechende Passung ein radiales Öffnen des Steckanschlusses am Verbindungsstift 6 verhindert.
3. Das abgestufte Schubteil 34 leitet die Bedienkraft als axiale Druckkraft an den Körper 52 der Kopfeinheit weiter.

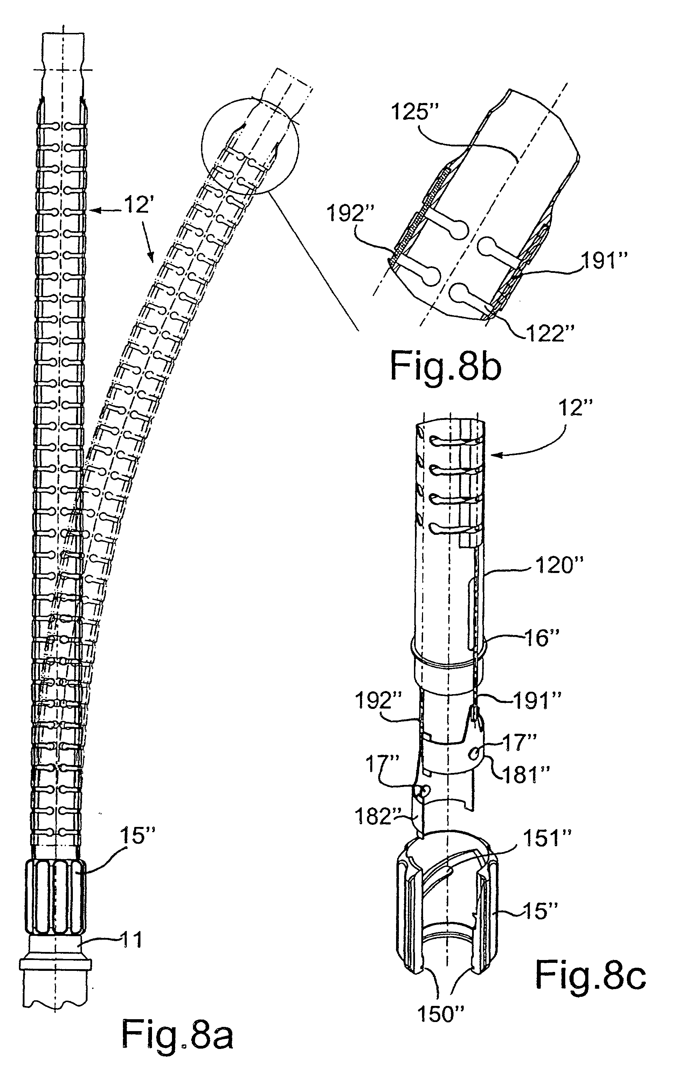

In Fig.8a ist ein in einer Ebene gesteuert auslenkbares Schaftrohr 12" dargestellt, und zwar in der mit ausgezogenen Linien wiedergegebenen Position in einer geraden Stellung und in einer gestrichelt wiedergegebenen Darstellung in einer in Fig.8a nach rechts ausgelenkten Position. Das in Fig.8a dargestellte Schaftrohr 12" kann anstelle eines beispielsweise in Fig.4 und 5 dargestellten, starren geraden Schaftrohrs 12 an dem sonst unveränderten Griffteil 2 montiert werden.

Das Verstellen in eine ausgelenkte Position ist beispielsweise mittels eines am proximalen Ende des Schaftrohrs 12" vorgesehenen Drehrings 15" durchführbar, welcher unmittelbar vor einer in Fig.8a nicht dargestellten Mutter 13 am Anschlußteil 11" und damit im Schaftteil integriert ist. Die Baugruppe Griffteil ist unverändert beibehalten.

Der Drehring 15" ist in einer in ihm ausgebildeten Nut 150" axial auf einem ringförmigen Ansatz 16" des Schaftrohrs 12" geführt und weist innen zwei gegenüberliegende Kulissen 151" auf, die mit vorzugsweise zylindrisch ausgebildeten, von Anschlußstücken 181", 182" vorstehenden Nasen 17" in Eingriff stehen. Durch Nasen 17" und gerade axiale Nuten 121" sind die Anschlußstücke 181" und 182" rotatorisch festgelegt.

Wird der Drehgriff 15" gedreht, so wird das eine Anschlußstück 181" in proximaler Richtung und das andere Anschlußstück 182" in distaler Richtung bewegt; beide Anschlußstücke 181" und 182" legen dann in entgegengesetzter Richtung die gleiche Strecke gegenüber dem unteren Ende 120" des Schaftrohrs 12" zurück. Dadurch wird das flexible Schaftrohr 12" in einem Bereich, in dem weitgehend beliebig verformbare Einkerbungen 122" zwischen Stegen 123" vorgesehen sind, elastisch und annähernd kreisbogenförmig verformt. Dabei behält die in Fig.8b strichpunktiert angedeutete, neutrale Faser 125" eine konstante Länge.

Ferner sind in Fig. 8c Zugmittel 191" und 192" angedeutet, die an ihren proximalen Enden mit den Anschlußstücken 181" bzw. 182" und mit ihren distalen Enden diametral gegenüberliegend an dem proximalen Ende des flexiblen Schaftrohrs 12" befestigt sind. (Siehe Fig.8b)

Eine in Fig.8a bis 8c nicht näher dargestellte Abdeckung des Schaftrohres 12" in Form beispielsweise eines schlauchartigen Überzugs dient dazu, die Einkerbungen 122' des Schaftrohrs abzudecken, wodurch ein nach außen und innen glattes Schaftrohr 12" geschaffen ist.

Der Drehring 15" ist zur Sicherung gegen ein unbeabsichtigtes Drehen und damit ein Auslenken des Schaftrohrs 2", was in Fig.8c nicht dargestellt ist, selbsthemmend ausgeführt oder ist mit einer ebenfalls nicht dargestellten Grenzkraftsperre versehen. Um die Montage zu erleichtern weisen die im Drehring 15" ausgebildeten, schräg verlaufenden und als Kulissen dienenden Nuten 151" eine in Fig.8c nicht dargestellte, vorzugsweise axial verlaufende Nutausführung auf.

### Bezugszeichenliste

- 1: Baugruppe Schaftteil
- 11: Anschlußteil
- 111: Anschlag für 13 und 26
- 112: Fläche von 11
- 1111: T-förmiges Fenster in 11
- 1112: schmaler Bereich
- 1113: breiter Bereich
- 115: Längsnut
- 116: ringförmige Anschlagfläche
- 117: rohrförmige Laufbahn
- 12: gerades Schaftrohr
- 12': einachsig gebogenes Schaftrohr
- 12": flexibles Schaftrohr
- 120": Ende von 12"
- 122': Einkerbung
- 123': Steg
- 13: Befestigungsmutter
- 14: Druckknopf-Anschluß für 5
- 15": Drehring
- 150": Nut
- 151": Nut
- 16": ringförmiger Ansatz
- 17": Nasen
- 181": Anschlußstück
- 182": Anschlußstück
- 191": Zugmittel
- 192": Zugmittel

- 2: Baugruppe Griffteil
- 21: Hebel
- 211: Sperre
- 212: Schenkelfeder
- 22: Schenkel
- 221: Bolzen
- 222: Querstift
- 223: Anschlag
- 251: Kontrollanzeige für Verstellmechanismus
- 252: Anzeigefenster "Feineinstellbereich"
- 253: Skala für Spaltbreite
- 26: Gehäuse
- 261: T- förmiges Fenster in 26
- 262: Gewinde
- 27: Montagenut
- 28: Lagerböcke
- 281: Aussparung
- 282: Gewinde
- 283: Bolzen

- 3: Baugruppe Mechanik
- 3: Baugruppe Mechanik
- 31: Anschlußteil für 32
- 311: Zylindrische Fläche von 31
- 312: Arbeitskanal
- 313: Nut
- 314: Nieten
- 315: Quernut
- 316: Längsnut
- 317: Hohlstift
- 318: Feder
- 3191: Anschlagfläche an 31
- 3192: Zylindrischer Abschnitt von 31
- 32: flexibles Schub- und Zugteil
- 321: Aussparungen von 32
- 322: Zylindrische Abschnitte von 32
- 323: Arbeitskanal
- 33: Kraftübertragende Metallkörper
- 34: abgestuftes Schubteil
- 341: Abschnitt von 34

- 4: Baugruppe Verstellmechanismus
- 41: Mittelteil von 4
- 411: Schlitz von 41
- 411a: Nut
- 412: Zylindrischer Ansatz
- 413: Bolzen
- 416: Gewindeeinsatz
- 417: Stirnfläche
- 42: Spindel
- 422: Fläche
- 423: Anschlag
- 425: Nut
- 43: Handrad
- 431: Verzahnung
- 436: Stift
- 44: Druckfeder
- 441: Absatz
- 45: Seele
- 453: Nieten
- 46: Anschluß
- 461: Umlaufende Nut in 46
- 47: Riegel
- 471: Verzahnung am Riegel 47

- 5: Klammermagazin
- 51: Schnappnase
- 52: Körper

- 6: Verbindungsstift

- 7: Andruckplatte

## Patentansprüche

1. Vorrichtung zum Betätigen von verschiedenenartigen, gegeneinander austauschbaren chirurgischen Instrumenten,
wobei am proximalen Ende eines Schaftteils (1) ein Griffteil (2) lagerichtig positioniert vorgesehen ist, an welchem ein Kniehebelmechanismus gehaltert ist, welcher mit dem proximalen Ende eines im Schaftteil (1) geführt untergebrachten, flexiblen kraftübertragenden Schub- und Zugteils (32, 45) in Wirkverbindung steht, dessen proximales Ende mit einem Verstellmechanismus (4) im Griffteil (2) verbunden ist, und an dessen distalem Ende ein Anschlussteil (46) zum Herstellen einer Verbindung zu einem chirurgischen Instrument vorgesehen ist, und
wobei am distalen Ende des Schaftteils (1) ein Anschluß (14) zum Anschließen eines Gehäuses eines chirurgischen Instruments vorgesehen ist, und am distalen Ende des flexiblen kraftübertragenden Schub- und Zugteils (32, 45) ein axiale Druckkraft an den Körper des chirurgischen Instruments übertragendes Schubteil angebracht ist, **dadurch gekennzeichnet, daß**
zum lösbaren Verbinden des Schaftteils (1) mit dem Griffteil (2) auf einem Anschlussteil (11) des Schaftteils (1) eine frei drehbare Befestigungsmutter (13) vorgesehen ist, wobei das Anschlussteil (11) einen gemeinsamen Anschlag (111) für Befestigungsmutter (13) und für Griffgehäuse (26) aufweist, und
der Kniehebelmechanismus aus einem zwischen Lagerböcken (28) schwenkbar gelagerten ersten Schenkel (21) und einem mit dem ersten Schenkel (21) beweglich verbundenen zweiten Schenkel (22) dadurch lösbar mit dem proximalen Ende des Schub- und Zugteils (32, 45) in Wirkverbindung steht, dass in einem Anschlussteil (31) am proximalen Ende des Schub- und Zugteils (32) eine aus Quer- und Längsnut gebildete Kreuznut (315, 316) vorgesehen ist, welcher im Griffgehäuse (26) und in dem Anschlussteil (11) der Kreuznut entsprechend ausgebildete T-förmige Fenster (261 bzw. 1111) zugeordnet sind.

2. Vorrichtung nach Anspruch 1, **dadurch gekennzeichnet, daß** zum Unterbinden eines unbeabsichtigten Betätigens des Kniehebelmechanismus (21, 22) in dessen ersten Schenkel (21) eine Sperre (211) mit Schenkelfeder (212) vorgesehen ist.

3. Vorrichtung nach Anspruch 1, **dadurch gekennzeichnet, daß** am rohrförmigen Griffgehäuse (26) eine Anzeige (222) zum Anzeigen der Lage des Verstellmechanismus (4) im freien Einstellbereich, eine Kontrollanzeige (251) bezüglich einer Arretierung des Verstellmechanismus (4) und eine Skala (253) zum Anzeigen der Einstellbreite eines Spaltes vorgesehen sind.

4. Vorrichtung nach Anspruch 1, **dadurch gekennzeichnet, daß** ein Mittelteil (41) des Verstellmechanismus (4) verdrehsicher im Schaftteil (1) geführt ist.

5. Vorrichtung nach Anspruch 4, **dadurch gekennzeichnet, daß** der Mittelteil (41) durch einen von ihm vorstehenden Bolzen (413) in einer Längsnut (115) des Schaftteils (1) verdrehsicher und durch einen kreiszylindrischen Ansatz (412) koaxial im Griffgehäuse (26) geführt ist.

6. Vorrichtung nach Anspruch 1, **dadurch gekennzeichnet, daß** zum Weiterleiten einer axial gerichteten Kraft entlang eines Schaftrohrs (12; 12') des Schaftteils (1) ein im Griffteil (2) koaxial geführtes Anschlußteil (31) durch zwei in dem flexiblen Schub- und Zugteil (32) eingebettete, steife Körper (33) mit diesem (32) fest verbunden ist.

7. Vorrichtung nach Anspruch 6, **dadurch gekennzeichnet, daß** die steifen Körper Metallkörper (33) sind.

8. Vorrichtung nach Anspruch 1, **dadurch gekennzeichnet, daß** ein mit dem Verstellmechanismus (4) verbundenes, kraftübertragendes Element (45) in einem vorzugsweise zentral angeordneten Arbeitskanal (312, 323) des flexiblen Schub- und Zugteils (32) untergebracht ist.

9. Vorrichtung nach Anspruch 8, **dadurch gekennzeichnet, daß** zum Verschieben des kraftübertragenden Elements (45) an dessen proximalen Ende der Mittelteil (41) des Verstellmechanismus (4) befestigt ist, der mittels einer in einem begrenzten Bereich rotatorisch bewegbaren und im Griffteil axial geführten Spindel (42) verschiebbar ist.

10. Vorrichtung nach Anspruch 9, **dadurch gekennzeichnet, daß** zum Übertragen einer Einstellbewegung als Bedienelement in dem Verstellmechanismus (4) ein mit der Spindel (42) in Wirkverbindung stehendes Handrad (43) vorgesehen ist.

11. Vorrichtung nach Anspruch 10, **dadurch gekennzeichnet, daß** zum Übertragen eines Bedienmoments von dem Handrad (43) auf die Spindel (42) ein in eine Nut (425) eingreifender Stift (436) vorgesehen ist.

12. Vorrichtung nach Anspruch 1, **dadurch gekennzeichnet, daß** zur Wegbegrenzung des axial verschiebbaren Schub- und Zugteils (32) ein Anschlag (3191) am Anschlußteil (31) sowie ein Anschlag (112) am Anschlußteil (111) ausgebildet sind.

13. Vorrichtung nach Anspruch 1, **dadurch gekennzeichnet, daß** das flexible Schub- und Zugteil (32) abwechselnd Aussparungen (321) und kreiszylindrische Abschnitte (322) zur Führung im Schaftrohr (12 bzw. 12') aufweist.

14. Vorrichtung nach Anspruch 1, **dadurch gekennzeichnet, daß** der Verstellmechanismus (4) zum Einstellen einer geforderten Spaltbreite dadurch arretierbar ist, daß eine Innenverzahnung (431) eines Handrades (4) unter Federwirkung (44) mit einer Außenverzahnung (471) eines mit dem Mittenteil (41) verbundenen Riegels (47) in Eingriff gebracht und gehalten wird.

15. Vorrichtung nach Anspruch 1, **dadurch gekennzeichnet, daß** ein Anschluß zum Anschließen eines chirurgischen Instruments am distalen Ende des Schaftrohrs (12', 12") als Druckknopfanschluß (14) mit an Biegelaschen vorgesehenen Druckknöpfen (51) ausgebildet ist.

16. Vorrichtung nach Anspruch 1, **dadurch gekennzeichnet, daß** zum Auslenken eines flexiblen Schaftteils (12") an dessen proximalem Ende diametral Zugmittel (191", 192") befestigt sind, deren entgegensetzte Enden an Anschlußstücken (181", 182") befestigt sind, die in einem verdrehbaren Hohlring (15") so geführt sind, daß bei Verdrehen des Hohlrings (15") die Anschlußstücke (181", 182") um die gleiche Strecke in entgegengesetzter Richtung verschoben werden.

17. Vorrichtung nach Anspruch 6, **dadurch gekennzeichnet, daß** in dem Hohlring (15") Führungskulissen (151") mit konstanter oder variabler Steigung ausgebildet sind, in denen von halbkreiszylindrischen Anschlußstücken (181", 182") vorstehende Nasen (17") aufgenommen sind.

## Claims

1. A device for actuating a variety of interchangeable surgical instruments, comprising at the proximal end of a shaft part (1) a handle part (2) positioned correctly located, a toggle mechanism actively connected to the proximal end of a flexible, force-communicating, reciprocating actuator (32, 45) guidingly accommodated in said shaft part (1), the proximal end of said flexible, reciprocating actuator (32, 45) being connected to a positioning mechanism (4) in said handle part (2), whereby provided at the distal end of said toggle mechanism is an adapter part (46) for producing a connection to a surgical instrument, comprising furthermore at the distal end of said shaft part (1) an adapter (14) for mounting a housing of a surgical instrument, whilst attached to the distal end of said flexible, reciprocating actuator (32, 45) is a pusher for communicating an axial compression force to said body of said surgical instrument, **characterized in that** for releasably connecting said shaft part (1) to said handle part (2) on an adapter part (11) of said shaft part (1) a freely rotatable fastener nut (13) is provided, said adapter part (11) comprising a common stop (111) for said fastener nut (13) and handle housing (26) and said toggle mechanism comprising a first leg (21) swivel-mounted between mounting pads (28) and a second leg (22) movably connected with said first leg (21) is actively connected to the proximal end of said reciprocating actuator (32, 45) **in that** in an adapter part (31) at the proximal end of said reciprocating actuator (32, 45) a cross-shaped groove, made up of a transverse groove (315) and longitudinal groove (316) is provided to which correspondingly configured T-shaped windows (261 and 1111 respectively) are assigned in said handle housing (269 and in said adapter part (11).

2. The device as set forth in claim 1, **characterized in that** to prevent accidental actuation of said toggle mechanism (21, 22) a lock (211) including leg spring (212) is provided in said first leg (21) thereof.

3. The device as set forth in claim 1, **characterized in that** provided on said tubular handle housing (26) are an indicator (222) for indicating the position of said positioning mechanism (4) in the free setting range, a checking indicator (251) indicating locking of said positioning mechanism (4) and a scale (253) indicating the setting width of a gap.

4. The device as set forth in claim 1, **characterized in that** a middle part (41) of said positioning mechanism (4) is guided non-rotatively in said shaft part (1).

5. The device as set forth in claim 4, **characterized in that** said said middle part (41) is guided non-rotatively in a longitudinal groove (115) of said shaft part (1) by a pin (413) protruding from said middle part (41) and coaxially in said handle housing (26) by a circular-cylindrical boss (412).

6. The device as set forth in claim 1, **characterized in that** for communicating an axial oriented force along a shaft tube (12; 12') of said shaft part (1) an adapter part (31) coaxially guided in said handle part (2) is fixedly connected to said flexible, reciprocating actuator (32) by two stiff bodies (33) embedded in said flexible, reciprocating actuator (32).

7. The device as set forth in claim 6, **characterized in that** said stiff bodies (33) are metal bodies.

8. The device as set forth in claim 1, **characterized in that** a force-communicating element (45) connected to said positioning mechanism (4) is accommodated in a preferably centrally arranged working passage (312, 323) of said flexible, reciprocating actuator (32).

9. The device as set forth in claim 8, **characterized in that** for shifting said force-communicating element (45) said middle part (41) of said positioning mechanism (4) is secured to the proximal end thereof which is shiftable by means of a spindle (42) rotatively movable in a defined range and axially guided in said handle part.

10. The device as set forth in claim 9, **characterized in that** for communicating a setting movement a handwheel (43) actively connecting said spindle (42) is provided as an operating element in said positioning mechanism (4).

11. The device as set forth in claim 10, **characterized in that** for communicating an operating moment of force from said handwheel (43) to said spindle (42) a pin (436) engaging a groove (425) is provided.

12. The device as set forth in claim 1, **characterized in that** for defining the travel of said axially shiftable reciprocating actuator (32) a stop (3191) is configured on said adapter part (31) and a stop (112) is configured on said adapter part (111).

13. The device as set forth in claim 1, **characterized in that** said flexible, reciprocating actuator (32) comprises an alternating train of recesses (321) and circular-cylindrical sections (322) for guidance in said shaft tube (12 or 12').

14. The device as set forth in claim 1, **characterized in that** said positioning mechanism (4) for setting a required gap width can be locked in place by an inner toothing (431) of a handwheel (4) engaging an outer toothing (471) of a latch (47) connecting said middle part (41) with the aid of a spring (44).

15. The device as set forth in claim 1, **characterized in that** an adapter for mounting a surgical instrument to the distal end of said shaft tube (12',12") is configured as a push-button adapter (14) including push-buttons (51) provided as flexible lugs.

16. The device as set forth in claim 1, **characterized in that** secured to the proximal end for deflecting a flexible shaft part (12") are diametral retraction means (191", 192") whose opposing ends are secured to said adapters (181", 182") guided in a rotative hollow ring (15") so that when said hollow ring (15") is rotated, said adapters (181", 182") are travelled by the same amount in the opposite direction.

17. The device as set forth in claim 6, **characterized in that** configured in said hollow ring (15") are guide links (151") of constant or variable pitch in which lugs (17") protruding from semicircular cylindrical adapters (181", 182") are mounted.

## Revendications

1. Dispositif pour manoeuvrer des instruments chirurgicaux de divers types et interchangeables,
dans lequel on prévoit à l'extrémité proximale d'une pièce de tige (1) une pièce de saisie (2) exactement positionnée sur laquelle est maintenu un mécanisme de levier à genouillère, lequel est en liaison active avec l'extrémité proximale d'une pièce de poussée et de retrait (32, 45) insérée et guidée dans la pièce de tige (1), flexible et transmettant les forces, dont l'extrémité proximale est liée à un mécanisme de régulation (4) dans la pièce de saisie (2) et sur l'extrémité distale duquel est prévue une pièce de raccordement (46) destinée à établir une liaison avec un instrument chirurgical et
dans lequel on prévoit à l'extrémité distale de la pièce de tige (1) un raccordement (14) destiné à raccorder un boîtier d'un instrument chirurgical et à l'extrémité distale de la pièce de poussée et de retrait (32, 45) flexible et transmettant des forces est appliquée une pièce de poussée transmettant au corps de l'instrument chirurgical l'effort de pression axial,
**caractérisé en ce qu'**on prévoit, pour assurer la liaison détachable de la pièce de tige (1) et de la pièce de saisie (2), un écrou de fixation (13) tournant librement sur une pièce de raccordement (11) de la pièce de tige (1), la pièce de raccordement (11) présentant une butée (111) commune à l'écrou de fixation (13) et au boîtier de saisie (26) et
le mécanisme de levier à genouillère, composé d'un premier montant (21) logé de manière à basculer entre des supports de palier (28) et d'un deuxième montant (22), lié de façon mobile au premier montant (21), est en liaison active détachable avec l'extrémité proximale de la pièce de poussée et de retrait (32, 45) du fait qu'on prévoit dans une pièce de raccordement (31) à l'extrémité proximale de la pièce de poussée et de retrait (32) une rainure en croix (315, 316), formée d'une rainure transversale et longitudinale, à laquelle sont associées des fenêtres (261 ou 1111) en T formées de façon correspondante dans le boîtier de saisie (26) et dans la pièce de raccordement (11) de la rainure en croix.

2. Dispositif selon la revendication 1 **caractérisé en ce que**, pour empêcher une manoeuvre involontaire du mécanisme de levier à genouillère (21, 22), on prévoit dans son premier montant (21) un blocage (211) avec un ressort de montant (212).

3. Dispositif selon la revendication 1 **caractérisé en ce qu'**on prévoit sur le boîtier de saisie (26) en forme de tube un affichage (222) pour afficher la position du mécanisme de régulation (4) dans la zone de réglage libre, un affichage de contrôle (251) concernant l'arrêt du mécanisme de régulation (4) et une échelle (253) pour afficher la plage de réglage d'une fente.

4. Dispositif selon la revendication 1 **caractérisé en ce qu'**une pièce médiane (41) du mécanisme de régulation (4) est guidée dans la pièce de tige (1) de façon à protéger de la torsion.

5. Dispositif selon la revendication 4 **caractérisé en ce que** la pièce médiane (41) est protégée de la torsion par une goupille (413) placée devant elle dans une rainure longitudinale (115) de la pièce de tige (1) et est guidée coaxialement dans le boîtier de saisie (26) par une saillie (412) cylindrique circulaire.

6. Dispositif selon la revendication 1 **caractérisé en ce que**, pour transmettre une force dirigée axialement le long d'un tube de tige (12 ; 12') de la pièce de tige (1), une pièce de raccordement (31) guidée coaxialement dans la pièce de saisie (2) par deux corps rigides enrobés dans la pièce de poussée et de retrait (32) flexible est liée solidement à celle ci (32).

7. Dispositif selon la revendication 6 **caractérisé en ce que** les corps rigides sont des corps métalliques.

8. Dispositif selon la revendication 1 **caractérisé en ce qu'**un élément (45) transmettant les forces lié au mécanisme de régulation (4) est introduit dans un canal opératoire (312, 323), de préférence placé en position centrale, de la pièce de poussée et de retrait (32) flexible.

9. Dispositif selon la revendication 8 **caractérisé en ce que**, pour pousser l'élément (45) transmettant les forces, est fixée à son extrémité proximale la pièce médiale (41) du mécanisme de régulation (4) qu'on peut déplacer au moyen d'une broche (42), mobile de façon rotative dans une zone limitée et guidée axialement dans la pièce de saisie.

10. Dispositif selon la revendication 9 **caractérisé en ce qu'**on prévoit un volant (43) en liaison active avec la broche (42) afin de transmettre un mouvement de réglage en tant qu'élément de commande dans le mécanisme de régulation (4).

11. Dispositif selon la revendication 10 **caractérisé en ce qu'**on prévoit une cheville (436) qui est en prise dans une rainure (425) afin de transmettre sur la broche (42) un couple de commande à partir du volant (43).

12. Dispositif selon la revendication 1 **caractérisé en ce qu'**on forme une butée (3191) sur la pièce de raccordement (31) ainsi qu'une butée (112) sur la pièce de raccordement (111) afin de limiter la course de la pièce de poussée et de retrait (32) axialement mobile.

13. Dispositif selon la revendication 1 **caractérisé en ce que** la pièce de poussée et de retrait (32) présente alternativement des évidements (321) et des segments (322) cylindriques circulaires destinés au guidage dans le tube de tige (12 ou 12').

14. Dispositif selon la revendication 1 **caractérisé en ce que** le mécanisme de régulation (4) destiné à régler une plage de fente demandée peut être arrêté **en ce qu'**un endentement intérieur (431) d'un volant (4) est mis et maintenu en prise, sous l'effet de ressort (44), avec un endentement extérieur (471) d'un verrou (47) lié à la pièce médiane (41).

15. Dispositif selon la revendication 1 **caractérisé en ce qu'**un raccordement pour raccorder un instrument chirurgical à l'extrémité distale du tube de tige (12', 12") est formé en tant que raccordement de boutons-poussoirs (14) avec des boutons-poussoirs (51) prévus sur des attaches souples.

16. Dispositif selon la revendication 1 **caractérisé en ce que**, pour l'élongation d'une pièce de tige (12") flexible, sont fixés diamétralement sur son extrémité proximale des moyens de traction (191", 192") dont les extrémités opposées sont fixées à des pièces de raccordement (181", 182") qui sont guidées dans un anneau creux (15") susceptible de torsion, de telle manière que les pièces de raccordement (181", 182") sont poussées selon le même trajet en direction opposée lorsqu'on tourne l'anneau creux (15").

17. Dispositif selon la revendication 6 **caractérisé en ce que** dans l'anneau creux (15") sont formées des coulisses de guidage (151") à inclinaison constante ou variable dans lesquelles sont reçus des tenons (17") proéminents sur les pièces de raccordement (181", 182") cylindriques circulaires.
